# EUROPÄISCHE PATENTSCHRIFT

(11) **EP 0 697 406 B1**
(45) Veröffentlichungstag und Bekanntmachung des Hinweises auf die Patenterteilung: **17.01.2001**
(21) Anmeldenummer: 95112441.1
(22) Anmeldetag: 08.08.1995
(51) Int. Cl.: C07D 239/30

(54) **Verfahren zur Herstellung von 4,6-Dichlor- oder 2,4,6-Trichlorpyrimidin aus 4,6-Dihydroxypyrimidin oder Barbitursäure**
Process for the preparation of 4,6-dichloro- or 2,4,6-trichloropyrimidine from 4,6-dihydroxypyrimidine or barbituric acid
Procédé pour la préparation de 4,6-chloro- ou 2,4,6-trichloropyrimidine à partir de 4,6-dihydroxypyrimidine ou de l'acide barbiturique

(30) Priorität: 19.08.1994 DE 4429466
(43) Veröffentlichungstag der Anmeldung: 21.02.1996
(73) Patentinhaber: BAYER AG, 51368 Leverkusen (DE)
(72) Erfinder: Steffan, Guido, Dr., D-51519 Odenthal (DE)

(56) Entgegenhaltungen:
- EP-A- 0 101 561
- DE-A- 1 933 784
- PORTER A.E.A. 'Diazines and Benzodiazines, in: "Comprehensive organic chemistry" (Sammes, P.G. ed.), Band 4' 1979 , PERGAMON PRESS LTD. , OXFORD * Seite 119 - Seite 120 *
- BROWN D.J. 'Pyrimidines and their benzo derivatives, in: "Comprehensive heterocyclic chemistry" (Katritzky, A.R. & Rees, C.W. eds), Band 3, Teil 2B' 1984 , PERGAMON PRESS LTD. , OXFORD * Seite 89 *
- BROWN D.J. ET AL. 'The pyrimidines, in: "The chemistry of heterocyclic compounds" (Taylor E.C. ed.); Band 52 ' Oktober 1994 , J. WILEY & SONS, INC. , NEW YORK * Seite 329 - Seite 339 *

## Beschreibung

Die vorliegende Erfindung betrifft ein verbessertes Verfahren zur Herstellung von 4,6-Dichlorpyrimidin aus 4,6-Dihydroxypyrimidin und von 2,4,6-Trichlorpyrimidin aus Barbitursäure.

Derartige Polychlorpyrimidine sind Zwischenprodukte zur Herstellung von Pflanzenschutzmitteln und Farbstoffen.

Bei bekannten Verfahren zur Herstellung von 4,6-Dichlorpyrimidin wird 4,6-Dihydroxypyrimidin mit Phosphoroxychlorid und Dimethylanilin oder Pyridin versetzt (siehe J. Chem. Soc. 1943, 574; J. Chem. Soc. 1951, 2214; Bull. Soc. Chim. France 1959, 741 und Khim.-Pharm. Zhurnal 8 (12), 28 (1974) - engl. Übersetzung S. 741).

Zur Aufarbeitung wird dabei zunächst überschüssiges Phosphoroxychlorid abgezogen und dann der Rückstand entweder auf Eis ausgetragen und das Produkt durch Extraktion und Kristallisation gewonnen oder einer Sublimation unterworfen, bei der das Produkt als Sublimat erhalten wird. Nachteilig bei diesem Verfahren ist, daß Dimethylanilin oder Pyridin in großen Mengen eingesetzt werden, sie aber nur mit erheblichem Aufwand zurückgewonnen und wiederverwendet werden können. Außerdem sind die Ausbeuten (maximal 81,1 % d.Th.) noch verbesserungsbedürftig. Schließlich ist eine wäßrige Aufarbeitung sehr aufwendig wegen der Entsorgung der gebildeten Abwässer und der Handhabung von Extraktionsmitteln. Eine Aufarbeitung durch Sublimation ist in technischem Maßstab ebenfalls sehr aufwendig, z.B. im Hinblick auf die einzusetzenden Apparate und der arbeitshygienischen Erfordernisse, um das Produkt aus dem Sublimator zu entfernen.

Eigene Versuche, die bekannten Verfahren durch den Einsatz geringerer Mengen Dimethylanilin vorteilhafter zu gestalten scheiterten, weil dann die Ausbeute von 4,6-Dichlorpyrimidin stark zurückgeht und die Bildung von Hochsiedern und Harzen stark zunimmt (siehe Beispiel 7).

Aus der DE-A 19 33 784 und der EP-A 101 561 sind Verfahren zur Herstellung von Tetrachlorpyrimidin bekannt, bei denen man Barbitursäure bzw. 5,5-Dichlorbarbitursäure in Phosphoroxychlorid mit Phosphortrichlorid und Chlor in Gegenwart eines tertiären Amins umsetzt. Nicht beschrieben ist dort die Herstellung von 4,6-Dichlor- und 2,4,6-Trichlorpyrimidin, der Zusatz von Phosphortrichlorid und Chlor nach der Umsetzung und die Abtrennung des Amins.

Es wurde nun ein Verfahren zur Herstellung von 4,6-Dichlor- und 2,4,6-Trichlorpyrimidin durch Umsetzung von 4,6-Dihydroxypyrimidin oder Barbitursäure bzw. deren tautomeren Ketoverbindungen mit überschüssigem Phosphoroxychlorid in Gegenwart eines tertiären Amins gefunden, das dadurch gekennzeichnet ist, daß man nach dieser Umsetzung
a) pro Äquivalent gegen Chlor ausgetauschte Hydroxylgruppen 0,75 bis 1,5 Mol Phosphortrichlorid und 0,7 bis 1,4 Mol Chlor so zufügt, daß stets ein Überschuß von Phosphortrichlorid gegenüber Chlor vorliegt und
b) bei vermindertem Druck nacheinander Phosphoroxychlorid und das hergestellte Polychlorpyrimidin über eine Kolonne abdestilliert oder die Schritte a) und b) in umgekehrter Reihenfolge durchführt, wobei im Falle b) nach a) vor dem Phosphoroxychlorid noch Phosphortrichlorid und im Falle a) nach b) nach Durchführung des Schrittes a) erneut gebildetes Phosphoroxychlorid abdestilliert wird und
c) den dann vorliegenden Destillationsrückstand mit einer starken Base versetzt und aus diesem Gemisch das eingesetzte tertiäre Amin wiedergewinnt, indem man
d) die obere Phase abtrennt und
e) destillativ reinigt.

Barbitursäure ist die Ketoform von 2,4,6-Trihydroxypyrimidin.

4,6-Dichlor- und 2,4,6-Trichlorpyrimidin wurden im Folgenden auch als Polychlorpyrimidin(e) bezeichnet; 4,6-Dihydroxypyrimidin und Barbitursäure auch als Polyhydroxypyrimidin(e).

4,6-Dihydroxypyrimidin ist eine bekannte Verbindung, die beispielsweise aus Malonsäurediamid und Ethylformiat zugänglich ist (J. Chem. Soc. 1951, 2214). Barbitursäure, Phosphoroxychlorid, Phosphortrichlorid und Chlor sind im Handel erhältlich.

Pro Mol Polyhydroxypyrimidin kann man beispielsweise 2,5 bis 12 Mol Phosphoroxychlorid einsetzen. Vorzugsweise beträgt diese Menge beim Einsatz von 4,6-Dihydroxypyrimidin 3,5 bis 5 Mol und beim Einsatz von Barbitursäure 4,5 bis 10 Mol.

Geeignete tertiäre Amine sind beispielsweise alkylierte Pyridine, alkylierte Imidazole, alkylierte Indole, N-dialkylierte Aniline und tertiäre N-Alkylamine. Bevorzugt sind alkylierte Pyridine, alkylierte Imidazole und alkylierte Indole. Besonders bevorzugt sind 2-Methyl-5-ethyl-pyridin, 1,2-Dimethylimidazol und 2,3,3-Trimethylindolenin.

Tertiäres Amin kann man z.B. in Mengen von 1 bis 5 Mol pro Mol Polyhydroxypyrimidin einsetzen. Vorzugsweise liegt diese Menge beim Einsatz von 4,6-Dihydroxypyrimidin bei 1,8 bis 2,2 Mol und beim Einsatz von Barbitursäure bei 2,7 bis 3,5 Mol.

Für die Umsetzung von Polyhydroxypyrimidinen mit Phosphoroxychlorid in Gegenwart von tertiären Aminen sind beispielsweise Temperaturen im Bereich 0 bis 120°C geeignet. Bevorzugt sind Temperaturen von 20 bis 100°C. Man kann dabei z.B. so verfahren, daß man Phosphoroxychlorid und das Polyhydroxypyrimidin vorlegt und das tertiäre Amin zudosiert. Dabei ist es vorteilhaft bei niedriger Temperatur mit der Zudosierung zu beginnen, z.B. bei 0 bis 30°C, bei höherer Temperatur die Zudosierung zu beenden, z.B. bei 60 bis 95°C und noch 1 bis 5 Stunden bei höheren Temperaturen nachzurühren, z.B. bei 80 bis 105°C.

Man kann auch so verfahren, daß man Phosphoroxychlorid und das tertiäre Amin vorlegt und das Polyhydroxypyrimidin zudosiert. Dabei ist es vorteilhaft, die Zudosierung bei 80 bis 105°C durchzuführen und bei dieser Temperatur noch 1 bis 5 Stunden nachzurühren.

Auch andere Arbeitsweisen und andere Temperaturen im Bereich 0 bis 120°C sind möglich.

Danach setzt man gemäß Stufe a) dem Reaktionsgemisch die oben angegebenen Mengen Phosphortrichlorid und Chlor so zu, daß stets ein Überschuß von Phosphortrichlorid gegenüber Chlor im Reaktionsgemisch vorliegt. Der Überschuß wird vorteilhaft so bemessen, daß im Reaktionsgemisch stets 0,3 bis 5 Gew.-%, vorzugsweise 0,5 bis 2 Gew.-% freies Phosphortrichlorid vorliegen. Man kann dabei so verfahren, daß man Phosphortrichlorid und Chlor gleichmäßig und mit einer kurzen Vorgabe von Phosphortrichlorid zudosiert. Die Zugabe von Phosphortrichlorid und Chlor kann z.B. bei 60 bis 105°C erfolgen. Nach beendeter Zugabe von Phosphortrichlorid und Chlor ist es vorteilhaft, bei 60 bis 105°C noch einige Zeit nachzurühren, z.B. 10 Minuten bis 3 Stunden.

Zur Aufarbeitung des Reaktionsgemisches gemäß Stufe b) wird zunächst bei vermindertem Druck über eine Kolonne fraktioniert destilliert. Der Druck kann anfänglich z.B. bei 500 bis 50 mbar liegen. Die Kolonne kann beispielsweise 5 bis 20 theoretische Böden aufweisen. Die Kolonne kann beispielsweise mit einem Rücklaufverhältnis von 0,8 bis 5:1 betrieben werden. Zunächst destilliert noch vorhandenes Phosphortrichlorid ab, meist ist das eine nur kleine Menge. Als nächste Fraktion folgt Phosphoroxychlorid, das wie das abdestillierte Phosphortrichlorid wiederverwendet werden kann. Dann ist es vorteilhaft, den Druck z.B. im Bereich 100 bis 5 mbar einzustellen und bei Sumpftemperaturen bis zu 160 bis 200°C das hergestellte Polychlorpyrimidin abzudestillieren. Man kann dieses in einer Fraktion auffangen.

Wenn man besonders reines Polychlorpyrimidin erhalten möchte, so ist es vorteilhaft, zunächst eine Hauptfraktion aufzufangen, die Polychlorpyrimidin einer Reinheit von über 97% enthält. Bei beginnender leichter Gelbfärbung des kondensierenden Destillats kann man dann noch einen Nachlauf auffangen, der Polychlorpyrimidin einer Reinheit von über 80 % enthält und ca. 5 bis 10 Gew.-% der Hauptfraktion ausmacht. Diesen Nachlauf kann man in die entsprechende Destillation bei der Aufarbeitung des nächsten Ansatzes zurückführen.

Man kann mit dem erfindungsgemäßen Verfahren, u.a. abhängig vom eingesetzten tertiären Amin, Polychlorpyrimidine, insbesondere 4,6-Dichlorpyrimidin in Ausbeuten von rund 80 bis nahezu 100 % erhalten. Mit den bevorzugt einzusetzenden tertiären Aminen liegen die Ausbeuten im Bereich 95 bis nahezu 100 %.

Man kann die Stufen a) und b) auch in umgekehrter Reihenfolge durchführen. Wenn man in der Reihenfolge b) → a) arbeitet, dann ist nach der Stufe a) erneut gebildetes Phosphoroxychlorid abzudestillieren.

Bei der Reihenfolge a) → b) erhält man ein Polychlorpyrimidin, das Spuren von Phosphoroxychlorid enthält. Bei umgekehrter Reihenfolge, also b) → a), erhält man ein Polychlorpyrimidin, das Spuren des tertiären Amins enthält. Man wird also die hier möglichen Reihenfolgen so wählen, daß man ein Polychlorpyrimidin erhält, dessen Verunreinigungen bei der weiteren Verarbeitung am wenigsten stören.

Polychlorpyrimidine, die praktisch keine Verunreinigungen mehr enthalten kann man erhalten, wenn man das in Stufe b) abdestillierte Polychlorpyrimidin einer zweiten Destillation unterwirft oder die Destillation zur Abtrennung des Polychlorpyrimidins als Rektifikation durchführt.

Der nach der Abtrennung des Polychlorpyrimidins verbleibende Destillationsrückstand, der als wesentlichen Bestandteil das Hydrochlorid des eingesetzten tertiären Amins enthält, wird nun gemäß Stufe c) mit einer starken Base versetzt. Geeignet sind beispielsweise konzentrierte wäßrige Lösungen von Alkalihydroxiden. Bezogen auf 1 Mol eingesetztes tertiäres Amin kann man z.B. 1 bis 2 Mol, z.B. 35 bis 55 gew.-%ige wäßrige Kaliumhydroxid- oder Natriumhydroxid-Lösungen einsetzen. Die starke Base wird dem Destillationsrückstand vorzugsweise bei solchen Temperaturen zugesetzt, bei denen dieser, - wenn u.U. auch nur noch schwer - rührbar ist. Häufig sind Temperaturen von 60 bis 100°C geeignet. Die Zugabe der starken Base kann relativ langsam, z.B. im Verlauf von 1/2 bis 5 Stunden und unter gutem Rühren erfolgen.

Anschließend wird die obere Phase abgetrennt (Stufe d)).

Nunmehr wird die obere Phase gemäß Stufe e) bei vermindertem Druck erneut destilliert. Der Druck kann beispielsweise 100 bis 10 mbar betragen. Während der Destillation wird vorzugsweise gerührt und die Sumpftemperatur bis zum Ende der Destillation auf beispielsweise 150 bis 200°C hochgeführt.

Bei dieser Destillation fällt ein 2-phasiges Destillat an. Die untere, wäßrige Phase enthält i.a. nur wenig tertiäres Amin, z.B. unter 1 Gew.-% des Einsatzes. Die obere, organische Phase enthält das zurückgewonnene tertiäre Amin, meist über 90 Gew.-% der Einsatzmenge, in einer Reinheit von über 95 %. Es kann nach Abstrippen von Spuren von Wasser wieder für die Umsetzung von Polyhydroxypyrimidinen mit Phosphoroxychlorid verwendet werden.

Bei der Destillation hinterbleibt eine braune Salzmasse, die durch Auflösen in Wasser aus dem Destillationsgefäß entfernt werden kann.

Bei der Reihenfolge d) → e) ist es vorteilhaft größere Mengen von Wasser einzubringen bzw. eine verdünntere starke Base zu verwenden, um zwei flüssige, feststofffreie, gut voneinander abtrennbare Phasen zu erhalten. Damit steigt zwar die Menge des anfallenden Abwassers, man kann jedoch in einfachen Apparaten arbeiten.

Das erfindungsgemäße Verfahren hat eine Reihe von Vorteilen, die es für eine Anwendung im technischen Maßstab besonders geeignet macht. So kann das eingesetzte tertiäre Amin wiedergewonnen und erneut verwendet werden. Die Ausbeuten an Polychlorpyrimidinen, insbesondere 4,6-Dichlorpyrimidin liegen beim Einsatz bestimmter tertiärer Amine bei nahezu 100 % d.Th., sonst sind sie gleich bis besser als bei bekannten Verfahren. Durch die nunmehr mögliche destillative Aufarbeitung entstehen sehr viel weniger und viel einfacher zu entsorgende Abfälle. Sublimationsapparaturen und Extraktionsmittel müssen nicht gehandhabt werden. Nicht nur das im Überschuß eingesetzte Phosphoroxychlorid, sondern auch das beim erfindungsgemäßen Verfahren neu aus Phosphortrichlorid gebildete Phosphoroxychlorid fällt in so reiner Form an, daß es beliebig weiterverwendet werden kann.

### Beispiele

### Beispiel 1

2680 g Phosphoroxychlorid und 460 g 4,6-Dihydroxypyrimidin (98 %ig) wurden gemischt und bei Raumtemperatur beginnend in 90 Minuten 980 g 2-Methyl-5-ethyl-pyridin unter Rühren zudosiert. Dabei wurde durch Kühlung die Temperatur auf maximal 80°C begrenzt. Dann wurde 1 Stunde bei 90°C und 1 Stunde bei 100°C nachgerührt. Nunmehr wurden bei 100°C im Verlauf von 1 Stunde simultan 1105 g Phosphortrichlorid und 570 g Chlor so eindosiert, daß stets 25 ml Phosphortrichlorid im Überschuß gegenüber dem eingeleiteten Chlor im Reaktionsansatz vorhanden waren. Es wurde 30 Minuten bei 100°C nachgerührt, dann über eine Kolonne mit 20 theoretischen Böden und einem von 1:1 auf 3:1 steigenden Rücklaufverhältnis bei 100 mbar zunächst eine geringe Menge Phosphortrichlorid und dann 3835 g Phosphoroxychlorid abdestilliert bis zu einer Sumpftemperatur von 130°C. Danach wurde das hergestellte 4,6-Dichlorpyrimidin über eine kurze Kolonne mit 3 theoretischen Böden bei 20 mbar bis zu einer Sumpftemperatur von 175°C abdestilliert, wobei zunächst 553 g eines Hauptlaufs (Reinheit 98,1 %ig; Ausbeute 91 % d.Th.) und dann bei beginnender Destillation des Amin-hydrochlorids (leicht gelbliches sirupöses Kondensat) 41 g eines Nachlaufs (Reinheit 88 %ig; Ausbeute 6 % d.Th.). Die Gesamtausbeute an 4,6-Dichlorpyrimidin betrug demnach 97 % d.Th.

Danach wurden bei 90°C dem dickflüssigen Destillationsrückstand im Verlaufe von 2 Stunden und unter gutem Rühren 800 g 50 %ige wäßrige Natronlauge zugesetzt. Unter kräftigem Rühren wurde dann bei 50 mbar und 170°C Sumpftemperatur bis zur Trockene destilliert. Das Destillat bestand aus 492 g einer wäßrigen Unterphase, die 0,5 Gew.-% des eingesetzten 2-Methyl-5-ethyl-pyridins enthielt und 932 g einer organischen Oberphase, die zu 98,8 Gew.-% aus 2-Methyl-5-ethyl-pyridin bestand, was 94 Gew.-% des Einsatzes entspricht. Der pulvrige salzartige Destillationsrückstand löste sich fast vollständig in Wasser.

### Beispiel 2

2680 g Phosphoroxychlorid wurden vorgelegt und unter Kühlung 980 g 2-Methyl-5-ethyl-pyridin zugegeben. Dann wurden 460 g 4,6-Dihydroxypyrimidin im Verlauf von 1 Stunde eindosiert. Die weitere Umsetzung und die Aufarbeitung erfolgten wie in Beispiel 1 beschrieben.

4,6-Dichlorpyrimidin wurde in nahezu 100 %iger Ausbeute erhalten, und von dem Amin wurden 88 Gew.-% des Einsatzes zurückgewonnen.

### Beispiele 3 bis 6

Es wurde verfahren wie bei Beispiel 1, jedoch wurde jeweils ein anderes tertiäres Amin eingesetzt und jeweils mehrere Ansätze durchgeführt.

### Beispiel 3:

2,3,3-Trimethylindolenin, Ausbeute an 4,6-Dichlorpyrimidin: 98 bis 100 % d.Th.

### Beispiele 4:

1,2-Dimethylimidazol, Ausbeute an 4,6-Dichlorpyrimidin: 97 bis 99 % d.Th.

### Beispiel 5:

N-Dimethylanilin, Ausbeute an 4,6-Dichlorpyrimidin 78 bis 88 % d.Th.

### Beispiel 6:

Tri-n-butylamin, Ausbeute an 4,6-Dichlorpyrimidin 75 bis 85 % d.Th.

### Beispiel 7: (zum Vergleich)

460 g Phosphoroxychlorid und 62 g N,N-Dimethylanilin wurden gemischt und in das Gemisch bei 100°C im Verlauf von 5 Stunden 116 g 4,6-Dihydroxypyrimidin (98 %ig) mit einer Schnecke eindosiert. Danach wurde bei 106 bis 128°C 8 Stunden nachgerührt. Das Reaktionsgemisch wurde mit 300 g Chlorbenzol verdünnt und auf 1,2 kg Eis ausgetragen. Die organische Phase wurde abgetrennt und zweimal mit je 100 ml Wasser gewaschen und anschließend fraktioniert destilliert. So werden 85,7 g 4,6-Dichlorpyrimidin (= 58 % d.Th.) erhalten.

### Beispiel 8:

2 680 g Phosphoroxychlorid wurden vorgelegt und unter Kühlung 980 g 2-Methyl-5-ethylpyridin zugegeben. Dann wurden 460 g 4,6-Dihydroxypyrimidin so eindosiert, daß die Temperatur 40°C nicht überstieg.

Nach einer halben Stunde Nachreaktion bei 40 bis 45°C wurde zunächst Phosphoroxychlorid, dann 4,6-Dichlorpyrimidin abdestilliert, wie im Beispiel 1 beschrieben.

Es wurden 1 390 g Phosphoroxychlorid und 595 g 4,6-Dichlorpyrimidin (Gehalt 98,2 %) erhalten, was einer Ausbeute von 98 % der Theorie entspricht.

Der Destillationsrückstand - im wesentlichen das Dichlorphosphorsäure-Salz des Methylethylpyridins - wurde bei von 80 auf 100°C ansteigender Temperatur mit 1 200 g Phosphortrichlorid, 600 g Phosphoroxychlorid und 570 g Chlor zur Reaktion gebracht. Danach wurden - wie in Beispiel 1 beschrieben - zunächst 100 g Phosphortrichlorid und dann 1 810 g Phosphoroxychlorid durch Destillation erhalten.

Der dickflüssige Destillationsrückstand wurde aufgearbeitet, wie im Beispiel 1 beschrieben.

Es wurden 902 g 2-Methyl-5-ethylpyridin zurückerhalten (92 % des Einsatzes).

### Beispiel 9:

128,0 g Barbitursäure wurden mit 700 g Phosphoroxychlorid verrührt. Dann wurden 242 g 2-Methyl-5-ethylpyridin im Laufe einer Stunde so zugetropft, daß die Temperatur 55°C nicht überstieg. Anschließend wurde je eine halbe Stunde bei 70, 80 und 90°C nachgerührt, worauf die Barbitursäure praktisch vollständig in Lösung gegangen war. Nach Zugabe von 80 g Phosphortrichlorid wurden 411 g Phosphortrichlorid und 215 g Chlor im Laufe von zwei Stunden bei Temperaturen von 90 bis 95°C simultan eindosiert. Anschließend wurde 30 Minuten bei 100°C unter Rückfluß nachreagiert.

Nach Ende der HCl-Entwicklung wurden über eine Kolonne mit 20 theoretischen Böden und einem von 1:1 auf 3:1 steigenden Rücklaufverhältnis von 100 mbar zunächst eine geringe Menge Phosphortrichlorid und dann bei 50 mbar 1 135 g Phosphoroxychlorid bis zu einer Sumfptemperatur von 135°C abdestilliert. Danach wurden bei 4 bis 5 mbar, einer Sumpftemperatur zwischen 96 und 140°C und einer Kopftemperatur zwischen 75 und 120°C durch Destillation über eine 5-Boden-Kolonne 172 g 2,4,6-Trichlorpyrimidin (98,1 %ig) erhalten, was einer Ausbeute von 96 % der Theorie entspricht.

Der dickflüssige Destillationsrückstand (316 g) wurde aufgearbeitet, wie im Beispiel 1 beschrieben. Es wurden 226 g 2-Methyl-5-ethylpyridin zurückgewonnen (93 % des Einsatzes).

## Patentansprüche

1. Verfahren zur Herstellung von 4,6-Dichlor- oder 2,4,6-Trichlorpyrimidin durch Umsetzung von 4,6-Dihydroxypyrimidin oder Barbitursäure bzw. deren tautomeren Ketoverbindungen mit überschüssigem Phosphoroxychlorid in Gegenwart eines tertiären Amins, dadurch gekennzeichnet, daß man nach dieser Umsetzung
a) pro Äquivalent gegen Chlor ausgetauschte Hydroxylgruppen 0,75 bis 1,5 Mol Phosphortrichlorid und 0,7 bis 1,4 Mol Chlor so zufügt, daß stets ein Überschuß von Phosphortrichlorid gegenüber Chlor vorliegt und
b) bei vermindertem Druck nacheinander Phosphoroxychlorid und das hergestellte Polychlorpyrimidin über eine Kolonne abdestilliert
oder die Schritte a) und b) in umgekehrter Reihenfolge durchführt, wobei im Falle b) nach a) vor dem Phosphoroxychlorid noch Phosphortrichlorid und im Falle a) nach b) nach Durchführung des Schrittes a) erneut gebildetes Phosphoroxychlorid abdestilliert wird und
c) den dann vorliegenden Destillationsrückstand mit einer starken Base versetzt und aus diesem Gemisch das eingesetzte tertiäre Amin wiedergewinnt, indem man
d) die obere Phase abtrennt und
e) destillativ reinigt.

2. Verfahren nach Anspruch 1, dadurch gekennzeichnet, daß man pro Mol Polyhydroxypyrimidin 2,5 bis 12 Mol Phosphoroxychlorid einsetzt.

3. Verfahren nach Ansprüchen 1 und 2, dadurch gekennzeichnet, daß man als tertiäres Amin ein alkyliertes Pyridin, ein alkyliertes Imidazol, ein alkyliertes Indol, ein N-dialkyliertes Anilin oder ein tertiäres N-Alkylamin in einer Menge von 1 bis 5 Mol pro Mol Polyhydroxypyrimidin einsetzt.

4. Verfahren nach Ansprüchen 1 bis 3, dadurch gekennzeichnet, daß man die Umsetzung des Polyhydroxypyrimidins mit Phosphoroxychlorid in Gegenwart eines tertiären Amins bei 0 bis 120°C durchführt und Phosphortrichlorid und Chlor bei 60 bis 105°C zufügt.

5. Verfahren nach Ansprüchen 1 bis 4, dadurch gekennzeichnet, daß man bei 500 bis 50 mbar Phosphortrichlorid und Phosphoroxychlorid und bei Drucken von 100 bis 5 mbar und bei Sumpftemperaturen bis zu 160 bis 200°C das Polychlorpyrimidin abdestilliert.

6. Verfahren nach Ansprüchen 1 bis 5, dadurch gekennzeichnet, daß man das im Überschuß eingesetzte und das neu gebildete Phosphoroxychlorid nach dessen Abtrennung beliebig weiter verwendet.

7. Verfahren nach Ansprüchen 1 bis 6, dadurch gekennzeichnet, daß man das Polychlorpyrimidin als Hauptfraktion und als Nachlauf auffängt.

8. Verfahren nach Ansprüchen 1 bis 7, dadurch gekennzeichnet, daß man als starke Base eine konzentrierte wäßrige Lösung von Alkalihydroxiden in einer Menge von 1 bis 2 Mol, bezogen auf 1 Mol eingesetztes tertiäres Amin, einsetzt.

9. Verfahren nach Ansprüchen 1 bis 8, dadurch gekennzeichnet, daß man bei 100 bis 10 mbar und Sumpftemperaturen bis zu 150 bis 200°C das eingesetzte tertiäre Amin abdestilliert und dabei als Destillat eine untere wäßrige und eine obere, das tertiäre Amin enthaltende Phase erhält.

## Claims

1. Process for the preparation of 4,6-dichloro- or 2,4,6-trichloropyrimidine by reaction of 4,6-dihydroxypyrimidine or barbituric acid or tautomeric keto compounds thereof with excess phosphorus oxychloride in the presence of a tertiary amine, characterized in that, in this reaction,
a) 0.75 to 1.5 mol of phosphorus trichloride and 0.7 to 1.4 mol of chlorine per equivalent of hydroxyl groups to be replaced by chlorine are added such that an excess of phosphorus trichloride over chlorine is always present and
b) phosphorus oxychloride and the polychloropyrimidine prepared are distilled off successively over a column under reduced pressure,
or steps a) and b) are carried out in the reverse sequence, phosphorus trichloride also being distilled off before the phosphorus oxychloride in the case of b) after a) and phosphorus oxychloride which has formed again after step a) has been carried out being distilled off in the case of a) after b), and
c) a strong base is added to the distillation residue which is then present, and the tertiary amine employed is recovered from this mixture by
d) separating off the upper phase and
e) purifying it by distillation.

2. Process according to Claim 1, characterized in that 2.5 to 12 mol of phosphorus oxychloride are employed per mole of polyhydroxypyrimidine.

3. Process according to Claims 1 and 2, characterized in that an alkylated pyridine, an alkylated imidazole, an alkylated indole, an N-dialkylated aniline or a tertiary N-alkylamine is employed as the tertiary amine in an amount of 1 to 5 mol per mole of polyhydroxypyrimidine.

4. Process according to Claims 1 to 3, characterized in that the reaction of the polyhydroxypyrimidine with phosphorus oxychloride in the presence of a tertiary amine is carried out at 0 to 120°C and phosphorus trichloride and chlorine are added at 60 to 105°C.

5. Process according to Claims 1 to 4, characterized in that the phosphorus trichloride and phosphorus oxychloride are distilled off under 500 to 50 mbar and the polychloropyrimidine is distilled off under pressures of 100 to 5 mbar and at bottom temperatures of up to 160 to 200°C.

6. Process according to Claims 1 to 5, characterized in that the phosphorus oxychloride employed in excess and that newly formed are further used as desired after removal thereof.

7. Process according to Claims 1 to 6, characterized in that the polychloropyrimidine is collected as the main fraction and as after-runnings.

8. Process according to Claims 1 to 7, characterized in that a concentrated aqueous solution of alkali metal hydroxides is employed as the strong base in an amount of 1 to 2 mol per mole of tertiary amine employed.

9. Process according to Claims 1 to 8, characterized in that the tertiary amine employed is distilled off under 100 to 10 mbar and at bottom temperatures of up to 150 to 200°C, and a lower, aqueous phase and an upper phase comprising the tertiary amine are obtained as the distillate.

## Revendications

1. Procédé pour la préparation de 4,6-dichloro- ou de 2,4,6-trichloropyrimidine par réaction de 4,6-dihydroxypyrimidine ou d'acide barbiturique respectivement de leurs composés céto tautomères avec de l'oxychlorure de phosphore en excès en présence d'une amine tertiaire, caractérisé en ce qu'après cette réaction
a) on ajoute par équivalent de groupes hydroxyle échangés avec le chlore de 0,75 à 1,5 moles de trichlorure de phosphore et de 0,7 à 1,4 moles de chlore de telle sorte qu'un excès de trichlorure de phosphore est toujours présent par rapport au chlore et
b) on élimine par distillation sur une colonne à pression réduite successivement l'oxychlorure de phosphore et la polychloropyrimidine préparée ou on réalise les étapes a) et b) dans l'ordre inverse, du trichlorure de phosphore étant encore éliminé par distillation avant l'oxychlorure de phosphore dans le cas b) suivant a) et de l'oxychlorure de phosphore formé à nouveau étant éliminé par distillation après la réalisation de l'étape a) dans le cas a) suivant b) et
c) on fait réagir le résidu de distillation alors présent avec une base forte et on récupère de ce mélange l'amine tertiaire utilisée, en ce que
d) on sépare la phase supérieure et
e) on la purifie par distillation.

2. Procédé selon la revendication 1, caractérisé en ce que l'on utilise par mole de polyhydroxypyrimidine de 2,5 à 12 moles d'oxychlorure de phosphore.

3. Procédé selon les revendications 1 et 2, caractérisé en ce que l'on utilise comme amine tertiaire une pyridine alkylée, un imidazole alkylé, un indole alkylé, une aniline N-dialkylée ou une N-alkylamine tertiaire dans une quantité de 1 à 5 moles par mole de polyhydroxypyrimidine.

4. Procédé selon les revendications 1 à 3, caractérisé en ce que l'on réalise la réaction de la polyhydroxypyrimidine avec de l'oxychlorure de phosphore en présence d'une amine tertiaire à de 0 à 120°C et en ce que l'on ajoute du trichlorure de phosphore et du chlore à de 60 à 105°C.

5. Procédé selon les revendications 1 à 4, caractérisé en ce que l'on élimine par distillation à de 500 à 50 mbar du trichlorure de phosphore et de l'oxychlorure de phosphore et la polychloropyrimidine à des pressions de 100 à 5 mbar et à des températures de queue allant jusqu'à de 160 à 200°C.

6. Procédé selon les revendications 1 à 5, caractérisé en ce que l'on réutilise de manière quelconque l'oxychlorure de phosphore utilisé en excès et l'oxychlorure de phosphore formé à nouveau après sa séparation.

7. Procédé selon les revendications 1 à 6, caractérisé en ce que l'on recueille la polychloropyrimidine comme fraction principale et comme queues de distillation.

8. Procédé selon les revendications 1 à 7, caractérisé en ce que l'on utilise comme base forte une solution aqueuse concentrée d'hydroxydes d'alcalis dans une quantité de 1 à 2 moles, rapportée à 1 mole d'amine tertiaire utilisée.

9. Procédé selon les revendications 1 à 8, caractérisé en ce que l'on élimine par distillation à de 100 à 10 mbars et à des températures de queue allant jusqu'à de 150 à 200°C l'amine tertiaire utilisée et on obtient ce faisant comme distillat une phase inférieure aqueuse et une phase supérieure contenant l'amine tertiaire.
